(19) Europäisches Patentamt

European Patent Office

Office européen des brevets

(11) **EP 1 046 388 B1**

(12) **FASCICULE DE BREVET EUROPEEN**

(45) Date de publication et mention
de la délivrance du brevet:
**04.05.2005 Bulletin 2005/18**

(51) Int Cl.[7]: **A61K 7/02**

(21) Numéro de dépôt: **00400674.8**

(22) Date de dépôt: **10.03.2000**

(54) **Composition cosmétique contenant des particules de résine de mélamine-formaldéhyde ou d'urée-formaldéhyde et ses utilisations**

Kosmetische Zusammensetzung enthaltend Melaminformaldehyd- oder Harnstoffformaldehydteilchen und ihre Anwendungen

Cosmetic composition containing melamine-formaldehyde or urea-formaldehyde particles and its uses

(84) Etats contractants désignés:
**AT BE CH CY DE DK ES FI FR GB GR IE IT LI LU MC NL PT SE**

(30) Priorité: **21.04.1999 FR 9905043**

(43) Date de publication de la demande:
**25.10.2000 Bulletin 2000/43**

(73) Titulaire: **L'OREAL**
**75008 Paris (FR)**

(72) Inventeurs:
• **Chevalier, Véronique**
**94440 Villecresnes (FR)**
• **Hurel, Valérie**
**91190 Gif-sur-Yvette (FR)**
• **Simon, Pascal**
**94400 Vitry-sur-Seine (FR)**

(74) Mandataire: **Rasson, Catherine**
**L'OREAL - D.I.P.I.**
**25-29 Quai Aulagnier**
**92600 Asnières (FR)**

(56) Documents cités:
**EP-A- 0 486 394       EP-A- 0 651 991**
**US-A- 5 219 561**

Remarques:
Le dossier contient des informations techniques présentées postérieurement au dépôt de la demande et ne figurant pas dans le présent fascicule.

EP 1 046 388 B1

## Description

La présente demande se rapporte à une composition notamment cosmétique contenant une phase huileuse et des particules de résine de mélamine-formaldéhyde ou d'urée-formaldéhyde, et à son utilisation comme composition de soin et/ou de maquillage de la peau, en particulier pour estomper les défauts du relief de la peau tels que les microreliefs, les rides, les pores, tout en conférant à celle-ci un aspect naturel, ainsi qu'à son utilisation pour le traitement des peaux grasses. La demande se rapporte aussi à l'utilisation de particules de résine de mélamine-formaldéhyde ou d'urée-formaldéhyde, comme agent matifiant dans une composition cosmétique.

Par définition, un produit matifiant est un produit qui empêche la peau de briller et qui unifie le teint. Les compositions de soin de la peau ou de maquillage ayant des propriétés matifiantes sont généralement utilisées pour résoudre les problèmes de brillance occasionnés par un excès de sébum et pour améliorer la tenue du maquillage à long terme qui a tendance à se dégrader visuellement au cours de la journée. Elles donnent un aspect mat à la peau résultant d'un pouvoir diffusant de la lumière à la surface de la peau. Elles peuvent aussi être utilisées pour estomper les défauts de la peau tels que les microreliefs, les rides, les ridules, les pores ou les variations de couleur.

Les compositions classiques dites matifiantes contiennent généralement des poudres absorbant le sébum et l'huile excédentaire de la composition non absorbée par la peau. Parmi les poudres matifiantes d'origine naturelle ou synthétique, on peut citer notamment les charges telles que le talc, l'amidon, le mica, la silice, les poudres de nylon, les poudres de polyéthylène, la poly-bêta-alanine, les poudres de poly(méth)acrylate de méthyle. Ce type de charges présente l'inconvénient de donner à la peau un aspect poudreux, pas naturel, qui peut même accentuer les défauts de la peau. De plus, les compositions les contenant sont généralement desséchantes à long terme et s'étalent difficilement. Leur effet matifiant est peu durable dans le temps.

Le document EP-A-0502769 décrit des compositions matifiantes apportant une couche translucide et un aspect naturel à la peau maquillée. Il s'agit de dispersions de particules sphériques dans un liant gras dans un rapport en poids charges/liant très spécifique. Pour avoir un effet matifiant, il faut une forte proportion de poudres et, de ce fait, ces compositions peuvent être desséchantes. En outre, elles ont tendance à pelucher lors de l'étalement et à donner un effet blanchissant à la peau en raison de la forte concentration en poudres.

Le brevet US 5219561 décrit une composition de poudre compacte contenant des microsphères creuses de polymère thermoplastique, notamment de polymère urée-formaldéhyde.

La demande EP-A-486394 décrit des poudres coulées cosmétiques contenant des microsphères creuses organiques ou minérales. Ces microsphères peuvent être notamment en polymère urée-formaldéhyde.

La demande EP-A-651991 décrit une composition de maquillage de la peau comprenant une fraction pulvérulente particulière dispersée dans un liant formé d'une émulsion. La fraction pulvérulente peut comprendre des microsphères creuses de polymère thermoplastiques, notamment en polymère urée-formaldéhyde.

Il subsiste donc le besoin d'une composition matifiante confortable lors de l'application et qui ne provoque aucune irritation ou dessèchement de la peau après application.

La demanderesse a maintenant trouvé une composition surmontant les inconvénients de l'art antérieur.

La demanderesse a découvert de manière surprenante que l'utilisation de particules de résine de mélamine-formaldéhyde ou d'urée-formaldéhyde dans une composition contenant une phase huileuse conférait à cette composition un aspect mat sur la peau, et ceci de façon prolongée dans le temps et même avec une proportion assez faible de particules. De plus, la composition obtenue est confortable et non desséchante pour la peau.

La présente invention a pour objet une composition contenant dans un milieu physiologiquement acceptable, au moins une phase huileuse et des particules d'au moins une résine choisie parmi les résines de mélamine-formaldéhyde et les résines d'urée-formaldéhyde.

On entend par milieu physiologiquement acceptable dans la composition de l'invention, un milieu non toxique et susceptible d'être appliqué sur la peau (y compris l'intérieur des paupières) ou les lèvres d'êtres humains.

La composition de l'invention grâce à la présence des particules de résine permet de donner un aspect mat à la peau. Aussi, l'invention a encore pour objet l'utilisation de particules d'au moins une résine choisie parmi les résines de mélamine-formaldéhyde et les résines d'urée-formaldéhyde, comme agent matifiant dans une composition cosmétique.

Les résines de mélamine-formaldéhyde et les résines d'urée-formaldéhyde utilisées selon l'invention se présentent sous forme d'une poudre dont les particules ont une dimension moyenne en nombre allant d'environ 0,1 à 20 μm selon les résines. Les particules de résine de mélamine-formaldéhyde ont généralement une dimension moyenne en nombre allant d'environ 0,1 à 0,5 μm et elles peuvent former des agglomérats allant jusqu'à 5 μm. Les particules de résine d'urée-formaldéhyde ont généralement une dimension moyenne en nombre allant d'environ 5 à 20 μm.

Comme particules de résine utilisables dans la composition de l'invention, on peut citer par exemple les résines de mélamine-formaldéhyde, commercialisées sous la dénomination MICROSILK MP par la société Grantec, sous la dénomination FLUIDIFIANT FL01 par la société Lafarge ou sous la dénomination CHRYSOGYPLAST PL100-R par la société Chryso, et les résines d'urée-formaldéhyde commercialisées sous les dénominations PERGOPAK, telle que PERGOPAK M3 par la société Martinswerk.

La quantité de particules de résine mélamine-formaldéhyde et/ou de résine d'urée-formaldéhyde dans la composition de l'invention dépend de l'effet recherché. Elle peut aller par exemple de 0,05 à 20 % et de préférence de 0,1 à 10 % en poids par rapport au poids total de la composition.

La nature de la phase huileuse de la composition de l'invention n'est pas critique, et la phase huileuse peut être constituée de tous les corps gras et notamment les huiles, classiquement utilisés dans le domaine cosmétique.

Parmi les huiles utilisables dans la composition de l'invention, on peut notamment citer par exemple les huiles d'origine végétale (jojoba, avocat, sésame, tournesol, maïs, soja, carthame, pépins de raisin), les huiles minérales (vaseline, isoparaffines éventuellement hydrogénées), les huiles de synthèse (myristate d'isopropyle, octanoate de cétéaryle, polyisobutylène, palmitate d'éthyl-hexyle, alkyl-benzoates), les huiles de silicone volatiles ou non volatiles telles que les polydiméthylsiloxanes (PDMS) et les cyclodiméthylsiloxanes ou cyclométhicones, notamment le cyclohexadiméthylsiloxane et le cyclopentadiméthylsiloxane, et les huiles fluorées ou fluorosiliconées. Les autres corps gras susceptibles d'être présents dans la phase huileuse peuvent être par exemple les acides gras, les alcools gras tels que l'alcool stéarylique, l'acool cétylique et l'alcool cétéarylique, et les cires.

La composition de l'invention peut se présenter sous forme d'un produit anhydre ou, en ajoutant au moins une phase aqueuse, sous forme d'une émulsion huile-dans-eau (H/E) ou eau-dans-huile (E/H) ou d'une émulsion multiple. On entend ici par "émulsion" aussi bien des dispersions sans émulsionnants que des dispersions comportant des émulsionnants ou encore des dispersions stabilisées par des particules solides ou par des sphérules lipidiques de type ionique ou non ionique. Pour une utilisation pour les peaux grasses, on préfère avoir une émulsion H/E.

Quand la composition selon l'invention est anhydre, la phase huileuse est généralement présente en une concentration allant de 60 à 99,9 % et de préférence de 80 à 99,9 % du poids total de la composition.

Dans les compositions de l'invention sous forme d'émulsion, la phase aqueuse de la composition peut être présente en une concentration allant par exemple de 5 à 80 % et de préférence 30 à 70 % en poids par rapport au poids total de la composition, et la phase huileuse peut être présente en une concentration allant de 5 à 70 % et de préférence de 10 à 50 % en poids par rapport au poids total de la composition.

Les émulsions peuvent contenir au moins un émulsionnant choisi parmi les émulsionnants amphotères, anioniques, cationiques ou non ioniques, utilisés seuls ou en mélange.

Les émulsionnants sont choisis de manière appropriée suivant l'émulsion à obtenir (E/H ou H/E).

Pour les émulsions H/E, on peut citer par exemple les émulsionnants suivants :

- comme émulsionnants amphotères, les N-acyl-aminoacides tels que les N-alkyl-aminoacétates et le cocoamphodiacetate disodique et les oxydes d'amines tels que l'oxyde de stéaramine ;

- comme émulsionnants anioniques, les acylglutamates tels que le "disodium hydrogenated tallow glutamate" (AMISOFT HS-21[R] commercialisé par la société Ajinomoto) ; les acides carboxyliques et leurs sels tels que le stéarate de sodium ; les esters phosphoriques et leurs sels tels que le "DEA oleth-10 phosphate" ; les sulfosuccinates tels que le "Disodium PEG-5 citrate lauryl sulfosuccinate" et le "Disodium ricinoleamido MEA sulfosuccinate" ;

- comme émulsionnants cationiques, les alkyl-imidazolidinium tels que l'éthosulfate d'isostéaryl-éthylimidonium ; les sels d'ammonium tels que le chlorure de N,N,N-triméthyl-1-docosanaminium (Behentrimonium chloride) ;

- comme émulsionnants non ioniques, les esters et éthers de sucres tels que le stéarate de sucrose, le cocoate de sucrose, et le mélange de stéarate de sorbitan et de cocoate de sucrose commercialisé par la société ICI sous la dénomination d'Arlatone 2121[R] ; les esters de polyol, notamment de glycérol ou de sorbitol, tels que le stéarate de glycéryle, le stéarate de polyglycéryl-2, le stéarate de sorbitan ; les éthers de glycérol ; les éthers oxyéthylénés et/ou oxypropylénés tels que l'éther oxyéthyléné, oxypropyléné de l'alcool laurique à 25 groupes oxyéthylénés et 25 groupes oxypropylénés (nom CTFA "PPG-25 laureth-25") et l'éther oxyéthyléné du mélange d'alcools gras en $C_{12}$-$C_{15}$ comportant 7 groupes oxyéthylénés (nom CTFA "C12-15 Pareth-7") ; les polymères d'éthylène glycol, tels que le PEG-100.

Pour les émulsions E/H, on peut citer par exemple comme émulsionnants les esters gras de polyol, notamment de glycérol ou de sorbitol, et notamment les esters isostéariques, oléiques et ricinoléiques de polyol, tels que le mélange de petrolatum, d'oléate de polyglycéryl-3, d'isostéarate de glycéryle, l'huile de ricin hydrogénée et d'ozokérite, vendu sous la dénomination PROTEGIN W[R] par la société Goldschmidt, l'isostéarate de sorbitan, le di-isostéarate de polyglycéryle, le sesquiisostéarate de polyglycéryle-2 ; les esters et éthers de sucres tels que le "Methyl glucose dioleate" ; les sels d'acide gras tels que le lanolate de magnésium ; les dimethicone copolyols et alkyl-dimethicone copolyols tels que le Laurylmethicone copolyol vendu sous la dénomination "Dow Corning 5200 Formulation Aid" par la société Dow Corning et le Cetyl dimethicone copolyol vendu sous la dénomination Abil EM 90[R] par la société Goldschmidt.

Les émulsionnants peuvent être introduits tels quels ou sous forme de mélange avec d'autres émulsionnants et/ou avec d'autres composés tels que des alcools gras ou des huiles.

La composition de l'invention peut contenir en plus, des adjuvants classiques tels que des colorants hydrosolubles ou liposolubles, des pigments, des parfums, des conservateurs, des filtres solaires, des séquestrants (EDTA), des actifs liposolubles ou hydrosolubles, des hydratants tels que les polyols et notamment la glycérine, des ajusteurs de pH (acides ou bases). Ces adjuvants sont utilisés dans les proportions habituelles dans le domaine cosmétique, et par exemple de 0,01 à 20 % en poids par rapport au poids total de la composition. Ces adjuvants ainsi que leurs concentrations doivent être tels qu'ils ne modifient pas la propriété recherchée pour la composition.

Comme actifs, on peut citer notamment les actifs utiles pour traiter les peaux grasses, tels que les sels de zinc et en particulier le gluconate de zinc ; les antibactériens comme l'acide salicylique, le triclosan, le lipacide, l'extrait de clou de girofle, l'octopirox, l'hexamidine ; les actifs anti-acné comme l'acide rétinoïque.

La composition de l'invention peut contenir en outre des charges en vue de modifier la texture de la composition. Comme charges qui peuvent être utilisées dans la composition de l'invention, on peut citer par exemple, outre les pigments, la poudre de silice ; le talc ; les particules de polyamide et notamment celles vendues sous la dénomination ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les poudres de matériaux organiques naturels tels que les amidons de maïs, de blé ou de riz, réticulés ou non, telles que les poudres d'amidon réticulé par l'anhydride octénylsuccinique, commercialisées sous la dénomination DRY-FLO par la société National Starch ; les microbilles de résine de silicone telles que celles commercialisées sous la dénomination TOSPEARL par la société Toshiba Silicone ; et leurs mélanges. Ces charges peuvent être présentes dans des quantités allant de 0 à 40% en poids et de préférence de 1 à 10 % en poids par rapport au poids total de la composition.

Bien que les particules utilisées dans la composition de l'invention aient un effet matifiant plus performant que celui des charges classiques, on peut, si on souhaite obtenir un effet couvrant important, avoir dans la composition de l'invention, une quantité totale de charges et de particules de résine, telle qu'elle soit égale ou supérieure à la concentration volumique C*. La concentration volumique C* dépend de la prise d'huile des charges, cette prise d'huile étant mesurée en déterminant le volume Vh de la fraction non volatile de la phase huileuse juste nécessaire pour combler les interstices entre les particules constituant les charges. La prise d'huile peut être mesurée par exemple selon la norme américaine ASTM D281-84.

Si V est le volume total des charges et V1 le volume de la fraction non volatile de la phase huileuse utilisée dans la composition, la concentration C* en % est égale à:

$$\frac{V}{V+Vh} \times 100$$

et la concentration volumique C des charges dans la composition considérée est égale à :

$$\frac{V}{V + V1} \times 100$$

On peut donc choisir les paramètres V et V1 tels que C soit au moins égale à C*.

Par ailleurs, selon la fluidité de la composition que l'on souhaite obtenir, on peut y ajouter un ou plusieurs gélifiants hydrophiles ou lipophiles, choisis par exemple parmi les argiles, les gommes polysaccharides et leurs dérivés (gomme de xanthane, carboxyméthylcellulose, hydroxypropylguar), les polymères carboxyvinyliques ou carbomers, les polyacrylamides tels que celui commercialisé sous la dénomination SEPIGEL 305 par la société Seppic et les polymères d'acide acrylamidométhylpropanesulfonique au moins partiellement réticulés tels que le produit commercialisé sous la dénomination HOSTACERIN AMPS par la société Hoechst. Ces gélifiants sont généralement utilisés à des concentrations allant de 0,1 à 10 %, de préférence 0,1 à 5 % et mieux de 0,1 à 3 % du poids total de la composition.

La composition de l'invention est destinée à une application topique et comprend de manière appropriée un milieu physiologiquement acceptable. Elle trouve notamment une application dans un grand nombre de traitements cosmétiques de la peau, et notamment en vue d'estomper les imperfections du relief de la peau, en particulier de camoufler les microreliefs, les rides et les ridules, les pores. Du fait de ses propriétés matifiantes, elle est également particulièrement appropriée pour le traitement des peaux grasses.

Aussi, un autre objet de l'invention consiste en l'utilisation cosmétique de la composition telle que définie précédemment, pour estomper les imperfections du relief de la peau et/ou pour camoufler les microreliefs, les rides, les ridules et/ou les pores de la peau.

L'invention concerne également un procédé de traitement cosmétique de la peau destiné à lui apporter un aspect mat et/ou à camoufler les défauts du relief de la peau, caractérisé par le fait qu'on applique sur la peau une composition telle que définie précédemment.

L'invention a encore pour objet l'utilisation de la composition telle que définie précédemment pour la préparation d'une composition destinée au traitement des peaux grasses.

Les exemples qui suivent servent à illustrer l'invention sans toutefois présenter un caractère limitatif. Les quantités y sont données en % en poids, sauf mention contraire.

**Exemple 1 : Emulsion huile dans eau**

*Phase huileuse :*

- Alcool stéarylique     1 %
- Mélange de stéarate de glycéryle et de PEG-100
  (Arlacel 165 de la société ICI Surfactants)     2 %
- Cyclohexadiméthylsiloxane     10 %

*Phase aqueuse :*

- Résine urée-formaldéhyde (Pergopak M3)     8 %
- Glycérine     5 %
- Carbomer     0,2 %
- Gomme de xanthane     0,2 %
- Hydroxyde de sodium     0,01 %
- EDTA     0,05 %
- Conservateurs     0,2 %
- Eau     qsp 100 %

<u>Mode opératoire</u> : on chauffe à 80°C la phase huileuse et la phase aqueuse sans la résine, puis on introduit la phase huileuse dans la phase aqueuse sous agitation et on incorpore ensuite la résine.

On obtient une composition matifiante apte à dissimuler les rides de façon naturelle.

**Exemple 2 : Emulsion huile dans eau**

*Phase huileuse :*

- Alcool stéarylique     1 %
- Mélange tartrate de dimyristyle/alcool cétéarylique/ C12-C15 Pareth-7/PPG-25 Laureth-25 (Cosmacol PSE de la société ENICHEM)     1,5 %
- Cyclohexadiméthylsiloxane     10 %

*Phase aqueuse :*

- Résine mélamine-formaldéhyde (Chrysogyplast PL100-R)     3 %
- Glycérine     5 %
- Carbomer     0,2 %
- Gomme de xanthane     0,2 %
- Hydroxyde de sodium     0,01 %
- EDTA (séquestrant)     0,05 %
- Conservateurs     0,2 %

- Aluminium Starch octenylsuccinate (Dry Flo de la société National Starch)     3 %
- Eau       qsp 100 %

Mode opératoire : on chauffe à 80°C la phase huileuse et la phase aqueuse sans la résine ni le Dry Flo, puis on introduit la phase huileuse dans la phase aqueuse sous agitation et on incorpore ensuite la résine et le Dry Flo.
On obtient une composition apte à matifier la peau en supprimant la brillance initiale.

**Exemple 3 : Emulsion huile dans eau**

*Phase huileuse :*

- Alcool stéarylique       1 %
- Mélange de stéarate de glycéryle et de PEG-100 (Arlacel 165 de la société ICI Surfactants)     2 %
- Cyclohexadiméthylsiloxane       10 %

*Phase aqueuse :*

- Résine mélamine-formaldéhyde (Chrysogyplast PL100-R)       2 %
- Glycérine       5 %
- Carbomer       0,2 %
- Gomme de xanthane       0,2 %
- Hydroxyde de sodium       0,01 %
- EDTA (séquestrant)       0,05 %
- Conservateurs       0,2 %
- Aluminium Starch octenylsuccinate (Dry Flo de la société National Starch)       3 %
- Eau       qsp 100 %

Le mode opératoire est le même que celui de l'exemple 2.
On obtient une composition matifiante apte à supprimer la brillance de la peau.

**Exemple comparatif :**

*Phase huileuse :*

- Alcool stéarylique       1 %
- Mélange de stéarate de glycéryle et de PEG-100 (Arlacel 165 de la société ICI Surfactants)     2 %
- Cyclohexadiméthylsiloxane       10 %

*Phase aqueuse :*

- Silice (Silica beads)       4 %
- Glycérine       5 %
- Carbomer       0,2 %
- Gomme de xanthane       0,2 %
- Hydroxyde de sodium       0,01 %
- EDTA       0,05 %
- Conservateurs       0,2 %
- Aluminium Starch octenylsuccinate (Dry Flo de la société National Starch)       3 %
- Eau       qsp 100 %

Test de matité : on a mesuré la matité obtenue pour les compositions de l'exemple 3 comprenant 2 % de résine utilisée selon l'invention et de l'exemple comparatif comprenant 4 % de silice. La mesure a été réalisée de la manière suivante : sur un support en caoutchouc, on a étalé la composition à raison de 2 g/cm$^2$, on a laissé sécher, puis on a mesuré la réflexion à l'aide d'un gonioréflectomètre, le résultat obtenu étant le rapport R entre la réflexion spéculaire et la réflexion diffuse.

| Composition | Exemple 3 | Exemple comparatif |
|---|---|---|
| R | 1,39 ± 0,075 | 1,37 ± 0,075 |

Ces résultats montrent qu'avec une concentration en résine deux fois moins importante, on obtient un résultat de matité identique à celui obtenu avec 4 % de silice.

## Revendications

**1.**  Composition contenant dans un milieu physiologiquement acceptable, au moins une phase huileuse et des particules d'au moins une résine choisie parmi les résines de mélamine-formaldéhyde et les résines d'urée-formaldéhyde, les particules de résines d'urée-formaldéhyde ayant une dimension moyenne en nombre allant de 0,1 à 20 μm.

**2.**  Composition selon la revendication 1, **caractérisée par le fait que** les particules de résine de mélamine-formaldéhyde ont une dimension moyenne en nombre allant de 0,1 à 20 μm.

**3.**  Composition selon la revendication 1 ou 2, **caractérisée par le fait que** la quantité de particules de résine va de 0,05 à 20 % en poids par rapport au poids total de la composition.

**4.**  Composition selon l'une quelconque des revendications précédentes, **caractérisée en ce qu'**elle est anhydre.

**5.**  Composition selon la revendication précédente, **caractérisée par le fait que** la phase huileuse représente de 60 à 99,9 % en poids par rapport au poids total de la composition.

**6.**  Composition selon l'une quelconque des revendications 1 à 3, **caractérisée par le fait qu'**elle se présente sous forme d'une émulsion.

**7.**  Composition selon la revendication précédente, **caractérisée par le fait que** la phase aqueuse représente de 5 à 80 % en poids par rapport au poids total de la composition.

**8.**  Composition selon la revendication 6 ou 7, **caractérisée par le fait que** la phase huileuse représente de 5 à 70 % en poids par rapport au poids total de la composition.

**9.**  Composition selon l'une quelconque des revendications 6 à 8, **caractérisée par le fait qu'**elle contient en outre au moins un émulsionnant.

**10.**  Composition selon l'une quelconque des revendications précédentes, **caractérisée par le fait qu'**elle contient en outre un ou plusieurs adjuvants choisis parmi les colorants, les pigments, les parfums, les conservateurs, les filtres solaires, les actifs liposolubles ou hydrosolubles, les séquestrants, les hydratants, les gélifiants, les charges.

**11.**  Composition selon la revendication précédente, **caractérisée en ce que** les charges sont choisies parmi la poudre de silice ; le talc ; les particules de polyamide ; les poudres de polyéthylène ; les microsphères à base de copolymères acryliques ; les poudres expansées ; les poudres de matériaux organiques naturels ; les microbilles de résine de silicone ; et leurs mélanges.

**12.**  Composition selon l'une quelconque des revendications précédentes, **caractérisée par le fait qu'**elle contient une quantité totale de charges et de particules de résine, égale ou supérieure à la concentration volumique C*.

**13.** Utilisation cosmétique de la composition selon l'une quelconque des revendications 1 à 12, pour estomper les imperfections du relief de la peau et/ou pour camoufler les microreliefs, les rides, les ridules et/ou les pores de la peau.

**14.** Procédé de traitement cosmétique de la peau, destiné à lui apporter un aspect mat et/ou à camoufler les défauts du relief de la peau, **caractérisé par le fait qu'**on applique sur la peau, une composition selon l'une quelconque des revendications 1 à 12.

**15.** Utilisation de la composition selon l'une quelconque des revendications 1 à 12 pour la préparation d'une composition destinée au traitement des peaux grasses.

**16.** Utilisation de particules d'au moins une résine choisie parmi les résines de mélamine-formaldéhyde et les résines d'urée-formaldéhyde, comme agent matifiant dans une composition cosmétique.

**Patentansprüche**

**1.** Zusammensetzung, die in einem physiologisch akzeptablen Medium mindestens eine Ölphase und Partikel mindestens eines Harzes enthält, das unter den Melamin-Formaldehyd-Harzen und Harnstoff-Formaldehyd-Harzen ausgewählt ist, wobei die Harnstoff-Formaldehyd-Harzpartikel eine zahlenmittlere Größe von 0,1 bis 20 µm aufweisen.

**2.** Zusammensetzung nach Anspruch 1, **dadurch gekennzeichnet, dass** die Melamin-Formaldehyd-Harzpartikel eine zahlenmittlere Größe von 0,1 bis 20 µm aufweisen.

**3.** Zusammensetzung nach Anspruch 1 oder 2, **dadurch gekennzeichnet, dass** der Mengenanteil der Harzpartikel im Bereich von 0,05 bis 20 Gew.-%, bezogen auf das Gesamtgewicht der Zusammensetzung, liegt.

**4.** Zusammensetzung nach einem der vorhergehenden Ansprüche, **dadurch gekennzeichnet, dass** sie wasserfrei ist.

**5.** Zusammensetzung nach dem vorhergehenden Anspruch, **dadurch gekennzeichnet, dass** die Ölphase 60 bis 99,9 Gew.-%, bezogen auf das Gesamtgewicht der Zusammensetzung, ausmacht.

**6.** Zusammensetzung nach einem der Ansprüche 1 bis 3, **dadurch gekennzeichnet, dass** sie in Form einer Emulsion vorliegt.

**7.** Zusammensetzung nach dem vorhergehenden Anspruch, **dadurch gekennzeichnet, dass** die wässerige Phase 5 bis 80 Gew.-%, bezogen auf das Gesamtgewicht der Zusammensetzung, ausmacht.

**8.** Zusammensetzung nach Anspruch 6 oder 7, **dadurch gekennzeichnet, dass** die Ölphase 5 bis 70 Gew.-%, bezogen auf das Gesamtgewicht der Zusammensetzung, ausmacht.

**9.** Zusammensetzung nach einem der Ansprüche 6 bis 8, **dadurch gekennzeichnet, dass** sie ferner mindestens einen Emulgator enthält.

**10.** Zusammensetzung nach einem der vorhergehenden Ansprüche, **dadurch gekennzeichnet, dass** sie außerdem einen oder mehrere Zusatzstoffe enthält, die unter den Farbstoffen, Pigmenten, Parfums, Konservierungsmitteln, Sonnenschutzfiltern, fettlöslichen oder wasserlöslichen Wirkstoffen, Maskierungsmitteln, Hydratisierungsmitteln, Gelbildnern und Füllstoffen ausgewählt sind.

**11.** Zusammensetzung nach dem vorhergehenden Anspruch, **dadurch gekennzeichnet, dass** die Füllstoffe unter Siliciumdioxidpulvern; Talk, Polyamidpartikeln; Polyethylenpulvern; Mikrokugeln auf der Basis von Acrylcopolymeren; expandierten Pulvern; Pulvern aus natürlichen organischen Materialien; Siliconharz-Mikrokugeln; und deren Gemischen ausgewählt sind.

**12.** Zusammensetzung nach einem der vorhergehenden Ansprüche, **dadurch gekennzeichnet, dass** sie eine Gesamtmenge an Füllstoffen und Harzpartikeln enthält, die der Volumenkonzentration C* entspricht oder darüber liegt.

**13.** Kosmetische Verwendung der Zusammensetzung nach einem der Ansprüche 1 bis 12, um die Unzulänglichkeiten des Hautreliefs zu mildern und/ oder die Mikroreliefe, Falten, Fältchen und/ oder Poren der Haut zu verdecken.

**14.** Verfahren zur kosmetischen Behandlung der Haut, das dazu dient, die Haut zu mattieren und/oder die Mängel des Hautreliefs zu verdecken, **dadurch gekennzeichnet, dass** auf die Haut eine Zusammensetzung nach einem der Ansprüche 1 bis 12 aufgetragen wird.

**15.** Verwendung der Zusammensetzung nach einem der Ansprüche 1 bis 12 zur Herstellung einer Zusammensetzung, die für die Behandlung von fettiger Haut vorgesehen ist.

**16.** Verwendung von Partikeln mindestens eines Harzes, das unter den Melamin-Formaldehyd-Harzen und Harnstoff-Formaldehyd-Harzen ausgewählt ist, als Mattierungsmittel in einer kosmetischen Zusammensetzung.

**Claims**

**1.** Composition comprising, in a physiologically acceptable medium, at least one oily phase and particles of at least one resin chosen from melamine-formaldehyde resins and urea-formaldehyde resins, the particles of urea-formaldehyde resins having a number-average size ranging from 0.1 to 20 $\mu$m.

**2.** Composition according to Claim 1, **characterized in that** the particles of melamine-formaldehyde resin have a number-average size ranging from 0.1 to 20 $\mu$m.

**3.** Composition according to Claim 1 or 2, **characterized in that** the amount of resin particles ranges from 0.05 to 20% by weight with respect to the total weight of the composition.

**4.** Composition according to any one of the preceding claims, **characterized in that** it is anhydrous.

**5.** Composition according to the preceding claim, **characterized in that** the oily phase represents from 60 to 99.9% by weight with respect to the total weight of the composition.

**6.** Composition according to any one of Claims 1 to 3, **characterized in that** it is provided in the form of an emulsion.

**7.** Composition according to the preceding claim, **characterized in that** the aqueous phase represents from 5 to 80% by weight with respect to the total weight of the composition.

**8.** Composition according to Claim 6 or 7, **characterized in that** the oily phase represents from 5 to 70% by weight with respect to the total weight of the composition.

**9.** Composition according to any one of Claims 6 to 8, **characterized in that** it additionally comprises at least one emulsifier.

**10.** Composition according to any one of the preceding claims, **characterized in that** it additionally comprises one or more adjuvants chosen from dyes, pigments, fragrances, preservatives, sunscreens, fat-soluble or water-soluble active principles, sequestering agents, moisturizers, gelling agents or fillers.

**11.** Composition according to the preceding claim, **characterized in that** the fillers are chosen from silica powder, talc; polyamide particles, polyethylene powders; microspheres based on acrylic copolymers; expanded powders; powders formed of natural organic materials; silicone resin microbeads; and their mixtures.

**12.** Composition according to any one of the preceding claims, **characterized in that** it comprises a total amount of fillers and of resin particles equal to or greater than the concentration by volume C*.

**13.** Cosmetic use of the composition according to any one of Claims 1 to 12 for softening imperfections of the relief of the skin and/or for concealing microrelief features, wrinkles, fine lines and/or pores of the skin.

**14.** Process for the cosmetic treatment of the skin intended to contribute a matt appearance to it and/or to conceal defects of the relief of the skin, **characterized in that** a composition according to any one of Claims 1 to 12 is applied to the skin.

15. Use of the composition according to any one of Claims 1 to 12 in the preparation of a composition intended for treating greasy skin.

16. Use of particles of at least one resin chosen from melamine-formaldehyde resins and urea-formaldehyde resins as mattifying agent in a cosmetic composition.